# EUROPEAN PATENT SPECIFICATION

(11) **EP 2 170 885 B1**
(45) Date of publication and mention of the grant of the patent: **06.10.2010**
(21) Application number: 08750103.7
(22) Date of filing: 06.05.2008
(51) Int. Cl.: C07D 471/04, A61K 31/437, A61P 35/00

(54) **BETA-CARBOLINE ARYLSULFONAMIDES, METHOD FOR PREPARING THEM AND COMPOSITION COMPRISING THEM**
BETA-CARBOLINARYLSULFONAMIDE, VERFAHREN ZU IHRER HERSTELLUNG UND ZUSAMMENSETZUNG, DIE SIE ENTHÄLT
BÊTA-CARBOLINE ARYLSULFONAMIDES, LEUR PROCÉDÉ DE PRÉPARATION ET COMPOSITION LES COMPRENANT

(30) Priority: 08.06.2007 IT MI20071163
(43) Date of publication of application: 07.04.2010
(73) Proprietor: C4T S.C. a.r.l., 00133 Roma (IT)
(72) Inventor: TOPAI, Alessandra, I-00153 Rome (IT); ROSSANO, Eugenio Claudio, I-83100 Avellino (IT); RECH, Francesca, 36077 Altavilla Vicentina (VI) (IT); BRECCIA, Perla, I-05010 Porano (TR) (IT); CERBARA, Ilaria, I-00177 Rome (IT); MARINI, Stefano, I-00173 Rome (IT); COLETTA, Massimiliano, I-00173 Rome (IT)
(74) Representative: Colombo, Stefano Paolo
(86) International application number: PCT/EP2008/055563
(87) International publication number: WO 2008/148617

(56) References cited:
- EP-A- 0 891 187
- WO-A-01/87883
- US-A- 6 066 633

## Description

### Technical field

The present invention relates to novel beta-carboline arylsulfonamides, to a method for preparing them and to a composition comprising them.

More particularly, the present invention relates to novel beta-carboline arylsulfonamides endowed with selective inhibitory activity towards matrix metalloproteases (MMP) involved in pathological conditions.

### Background of the invention

MMPs are zinc proteases whose substrates are the macromolecules that compose the extracellular matrix, and also a series of other molecules related thereto (McCawley et al. Curr Opin Cell Biology. 2001, 13, 534-540).

MMPs thus participate in all physiological processes involving the renewal and remodelling of tissues, such as pregnancy, embryo development, growth and cicatrization phenomena, angiogenesis, neurogenesis, inflammatory processes and apoptosis (Nagase et al. J. Biol Chem. 1999, 274, 21491-21494).

Numerous studies have also demonstrated their involvement in various pathological conditions; in particular, an increase in the level of certain MMPs has been recorded in the case of cardiovascular pathologies (Tayebjee et al. Curr Med Chem. 2005, 12, 917-925; Janicki et al. Heart Failure Reviews. 2004, 9, 33-42), in the central nervous system (Gu et al. J Neuroscience. 2005, 25, 6401-6408), in osteoarthritis and in rheumatoid arthritis (Wieland et al. Nature Rev Drug Discov. 2005, 4, 331-344), and in diseases of the oral cavity such as periodontitis (Sorsa et al. Oral diseases. 2004, 10, 311-318).

However, the majority of the studies relate to the role played by the various MMPs in the angiogenic process and, in particular, in tumour development. The start of the angiogenic process corresponds to degradation of the basal membrane subtending the blood vessel (Kalluri et al. Nat Rev Cancer. 2003, 3, 422-433), this degradation taking place by means of the action of proteolytic enzymes, in particular gelatinases MMP2 and MMP9 produced by endothelial and stromal cells, and, in the case of tumour pathologies, also by cancer cells (Coussens et al. Chem Biol. 1996, 3, 895-904).

Given their undisputed involvement in numerous pathologies, MMPs have for the last 20 years represented and continue to represent an objective for the development of numerous synthetic inhibitors.

Although many preclinical studies have given promising results regarding the use of MMP inhibitors in pathologies such as tumours and arthritis, the majority of the clinical experiments were abandoned due to lack of efficacy or to the occurrence of a serious musculoskeletal syndrome (Zucker et al. Oncogene. 2000. 19, 6642-6650; Coussens et al. Science. 2002, 295, 2387-2392).

Among the probable causes of failure of the clinical experiments, Overall and Kleifeld demonstrated the lack of selectivity (Overall et al. Nature Rev Cancer. 2006, 6, 227-239). Specifically, MMPs are capable of influencing tumour development not only via degradation of the extracellular matrix, as was initially thought (Liotta et al. Nature. 1980, 284, 67-68), but also via various signalling functions (McCawley et al. Curr Opin Cell Biol. 2001, 13, 534-540) which explain a series of protective activities with regard to metastatic growth (Bergers et al. Science. 1999, 284, 808-812; Epstein et al. Nature Rev Cancer. 2004, 4, 540-550).

The strategy of a broad-spectrum inhibition has, therefore, fallen into disuse (BB-2516-marimastat, AG-3340-prinomastat).

The need thus arose to find inhibitors that are selective for the MMPs most commonly involved in these pathologies. However, this objective is particularly difficult to achieve given the considerable homology of the catalytic sites of this family of proteases.

The majority of the synthetic inhibitors have, as a common functional group, hydroxamate (CONHOH), which is fundamental for the catalytic zinc-chelating action (Rao et al. Current Pharmaceutical Design. 2005, 11, 295-322), connected in various ways to a substituent capable of ensuring the interaction with the residues of the accessory "pockets" present in the catalytic site.

In particular, the region that is mainly discriminating between the various MMPs appears to be the S1' pocket (Whittaker et al. Chem Rev. 1999, 99, 2735-2776). MMP2, 3, 8, 9, 12 and 13 have a particularly long S1' pocket capable of accommodating bulkier and more hydrophobic residues when compared with MMP1 and 7, which, in position 197, have residues such as arginine and tyrosine that make it shorter and more encumbered.

The literature describes numerous beta-carboline derivatives of both natural and synthetic origin, which are endowed with pharmacological activity: from antitumour action (W02004/113336; Bioorg Med Chem. 2006, 14, 6998-7010; US 2003/0040527) to the treatment of erectile dysfunction (tadalafil, Daugan et al. J Med Chem. 2003, 46, 4525-4532).

The compounds described in document US 6 066 633A have a hydroxamic group in position 3 and a sulfonyl group on the nitrogen in position 2 of the tetrahydro-beta-carboline nucleus; these compounds have good inhibitory power with respect to MMP2, MMP3 and MMP9, but little selectivity with respect to the various isoforms.

The compounds described in document EP 0 891 187 B1 contain a carboxylic group in position 1 and/or 3; inhibitory activity on MMP8 alone has been demonstrated for these compounds.

However, as already pointed out, recent literature data (reviewed in Overall et al. Nature Rev Cancer. 2006, 6, 227-239; Peterson T. Cardiovascular Research. 2006, 69, 677-687) shows that selectivity has become a fundamental requirement for the development of a novel MMP inhibitor, in which the term "selectivity" in particular means the capacity for discriminating between MMPs involved in pathological processes and, thus, the "targets" and MMPs that play a key role in normal cell and tissue functioning and, thus, the "antitargets".

Literature sources show that they are targets for various pathologies:
- MMP2 in tumours, both for its collagen IV-cleaving capacity (Stetler-Stevenson. Invasion Metastasis. 1994, 14, 259-268) and for its proteolytic action with respect to chemokines that regulate the inflammatory response (McQuibban et al. Science 2000, 289, 1202-1206);
- MMP9 in central nervous system pathologies such as cerebral ischemia (Lipton et al. J Neuroscience. 2005, 25, 6401-6408);
- MMP13 in post-infarction remodelling (Wilson E.M., et al. Circulation. 2003, 107, 2857-63) and, together with MMP12, also in plaque instability (Johnson J.L. et al. PNAS. 2005, 102, 15575-80).

MMP3 (McCawley et al. Cancer Res. 2004, 64, 6965-6972), MMP1 and MMP14 are, however, antitargets: in particular, the appearance of musculoskeletal anomalies associated with the prolonged administration of broad-spectrum inhibitors is attributed to the inhibition of these metalloproteases (Fingleton B. Expert Opin Ther Targets. 2003, 7, 385-397; Holmbeck et al. Cell. 1999, 99, 81-92).

There is therefore still very much seen to be a need to find inhibitors that are selective towards the MMPs more involved in pathological conditions.

A novel family of beta-carboline compounds has now been found, which (a) are substituted with a carboxylic or hydroxamic group in position 1 and with a sulfonamide group in position 2 of the tetrahydro-beta-carboline nucleus, and (b) have inhibitory activity with respect to the target matrix metalloproteases MMP2, 8, 9, 12 and 13 and are discriminating with respect to the antitarget matrix metalloproteases MMP1, 3 and 14.

An important structural difference of the compounds of the present invention relative to the known MMP inhibitors is the presence of a sulfonamide group.

### Description of the invention

In the course of the present description and in the claims
- the abbreviation "MMP" is used to indicate matrix metalloproteases;
- the term "targets" is used in relation to MMPs involved in pathological processes; typical examples of target MMPs according to the present invention are MMP2, 8, 9, 12 and 13;
- the term "antitargets" is used in relation to MMPs that have a key role in normal cell and tissue functioning; typical examples of antitarget MMPs according to the present invention are MMP1, 3 and 14;
- the abbreviation "ZBG" is used to indicate the group that binds to zinc (zinc binding group).

The present invention relates to a beta-carboline arylsulfonamide that is endowed with inhibitory activity with respect to target MMPs and is discriminating with respect to antitarget MMPs; it is therefore useful in the treatments of pathologies associated with the overexpression of target MMPs.

In particular, the present invention relates to an arylsulfonamide of formula (I) 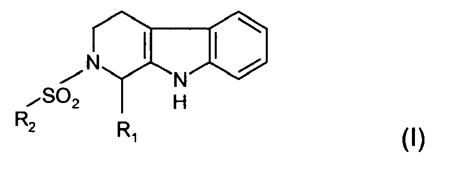 in which
R₁ is a carboxylic or hydroxamic group, and
R₂ is an aryl group chosen from the class comprising
- a naphthyl group and
- a phenyl group optionally substituted in the para position with
   (a) a halogen atom,
   (b) a linear or branched alkyl group containing from 1 to 5 carbon atoms,
   (c) an alkoxy group in which the linear or branched alkyl group contains from 1 to 5 carbon atoms,
   (d) a phenyl group which, in turn, is optionally substituted in the para position with a halogen atom, a linear or branched alkyl group containing from 1 to 5 carbon atoms, or an alkoxy group in which the linear or branched alkyl group contains from 1 to 5 carbon atoms, or
   (e) a phenoxy group in which the phenyl group is optionally substituted in the para position with a halogen atom, a linear or branched alkyl group containing from 1 to 5 carbon atoms or an alkoxy group in which the linear or branched alkyl group contains from 1 to 5 carbon atoms;
   stereoisomers thereof and, when R₁ is a carboxylic group, pharmaceutically acceptable base-addition salts thereof.

Preferred meanings of halogen are chlorine and fluorine.

Typical examples of R₂ are phenyl, 2-naphthyl, 4'-methylphenyl, 4'-tert-butylphenyl, 4'-butoxyphenyl, biphenyl, 4"-methoxy-4'-phenoxyphenyl, 4"-chloro-4'-phenoxyphenyl and 4"-fluoro-4'-phenoxyphenyl.

In a second aspect, the present invention relates to a process for preparing an arylsulfonamide of formula (I) 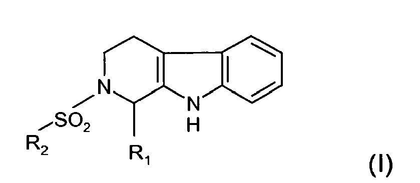 in which R₁ and R₂ have the meanings given above, and, when R₁ is a carboxylic group, the pharmaceutically acceptable base-addition salts thereof, characterized in that:
(a)
   (i) a compound of formula (II) 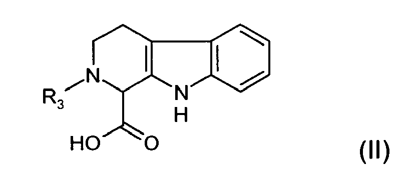 in which R₃ is a protective group,
      is immobilized on a suitable acid-labile solid support,
   (ii) the protective group R₃ is removed,
   (iii) the deprotected product is reacted with a sulfonyl chloride of formula (III)

      R₂-SO₂Cl (III)

      in which R₂ has the meanings given above,
      in the presence of a suitable acid acceptor,
      to give a compound of formula (I) in which R₁ is a carboxylic group,
   (iv) the compound of formula (I) thus obtained is removed from the solid support, and
   (v) if desired, the compound of formula (I) is salified with a pharmaceutically acceptable base, or
(b)
   (i) a compound of formula (II) is fluorinated to give a compound of formula (IV) 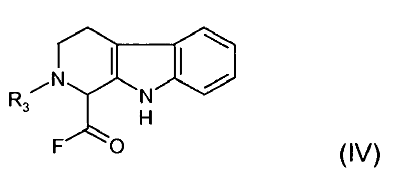 in which R₃ has the meaning given above,
   (ii) the compound of formula (IV) thus obtained is immobilized on a suitable solid support,
   (iii) the protective group R₃ is removed,
   (iv) the deprotected product is reacted with a sulfonyl chloride of formula (III)

      R₂-SO₂-Cl (III)

      in which R₂ has the meanings given above in the presence of a suitable acid acceptor,
   (v) the product thus obtained is removed from the solid support with NH₂OH to give a compound of formula (I) in which R₁ is a hydroxamic group.

Preferably, the protective group R₃ is the fluorenylmethoxycarbonile (Fmoc) group.

Advantageously, the acid-labile solid support used in step (a)(i) is a chlorotrityl resin.

In one preferred embodiment, the immobilization of the compound of formula (II) on the chlorotrityl resin is performed, in step (a)(i), in the presence of diisopropylethylamine (DIEA).

Preferably, the removal of the Fmoc protective group in steps (a)(ii) and (b)(iii) is performed with piperidine at 20% in dimethylformamide (DMF).

Preferably, the reaction of step (a)(iii) is performed by reacting the deprotected product with a moderate excess of sulfonyl chloride of formula (III). Advantageously, the ratio in equivalents is about 1 : 3 and the reaction is performed in the presence of about 2 equivalents of 4-dimethylaminopyridine (DMAP).

Advantageously, step (a)(iv) is performed using an acetic acid (AcOH)/trifluoroethanol (TFE)/dichloromethane (DCM) 1:1:8 mixture.

Preferably, the solid support used in step (b)(ii) is a hydroxymethyl resin. Advantageously, the hydroxymethyl resin NovaSynTGOH is used.

In one preferred embodiment, the immobilization of the compound of formula (IV) on the hydroxymethyl resin is performed, in step (b)(ii), in the presence of 4-dimethylaminopyridine (DMAP).

Typically, the reaction of step(b)(iv) is performed by reacting the deprotected product with an excess of sulfonyl chloride of formula (III). Advantageously, the ratio in equivalents is about 1 : 7 and the reaction is performed in the presence of 4-dimethylaminopyridine (DMAP; 0.01 equivalents).

Finally, step (b)(v) is preferably performed with NH₂OH as a 50% aqueous solution.

The compounds of formula (I) according to the present invention have, on the one hand, inhibitory power with respect to target MMPs (MMP2, MMP8, MMP9, MMP12 and MMP13) that is greater than or at least comparable to that of the previously known beta-carboline derivatives, and, on the other hand, are entirely novel with respect to the said beta-carboline derivatives since they show, unexpectedly, considerable discriminating capacity with respect to MMP1, MMP3 and MMP14 (proteases with a fundamental physiological role and therefore antitargets).

The simultaneous presence of the carboxylic or hydroxamic group ZBG (zinc-binding group) in position 1 and of the sulfonamide group in position 2 of the beta-carboline nucleus thus appears to have a synergistic effect on the selectivity with respect to the target MMPs. For example, the compound of Example 13 below [(1RS)-2-(4"-chloro-4'-phenoxybenzenesulfonyl)-1,2,3,4-tetrahydro-norarmano-N-hydroxy-1-carboxamide] shows, in point of fact, IC₅₀ values of less than 0.3 nM (intrinsic limit of the enzymatic test used) on MMP2; equal to 0.6 nM, 250 nM, 2 nM, 12 nM, 70 nM and 30 nM, respectively, on MMP3, 8, 9, 12, 13 and 14, and greater than 50 µM for MMP1. Even considering the limit value of 0.3 nM as given for MMP2, the decrease in inhibitory power with respect to the *antitarget* MMPs is therefore about 16 000-fold with respect to MMP1, 830 with respect to MMP3 and 100 with respect to MMP14, respectively (Table 1).

A third subject of the present invention thus consists of a pharmaceutical composition comprising a compound of formula (I) according to the present invention and at least one physiologically acceptable vehicle.

As reported in the literature, the *target* MMPs are involved in pathological conditions such as rheumatoid arthritis, arthrosis, atherosclerosis and tumours, including the dissemination and formation of metastasis.

Thus, typical examples of pathological conditions that may draw benefit from treatment with a pharmaceutical composition according to the present invention are rheumatoid arthritis, arthrosis, atherosclerosis and tumours.

Preferably, the pharmaceutical compositions of the present invention are prepared in suitable dosage forms comprising an effective dose of at least one compound of formula I and at least one physiologically acceptable inert ingredient suitable for oral, rectal, topical, intravenous, subcutaneous, intramuscular or intraperitoneal administration.

Examples of suitable dosage forms include tablets, capsules, coated tablets, granules, solutions and syrups for oral administration; creams, ointments and antiseptic plasters for topical administration; suppositories for rectal administration and sterile solutions for injection-mediated, aerosol or ophthalmic administration.

The dosage forms may also contain other traditional ingredients such as: preserving agents, stabilizers, surfactants, buffers, osmotic pressure-regulating salts, emulsifiers, sweeteners, colorants, flavourings and the like.

If required for particular therapies, the pharmaceutical composition of the present invention may contain other pharmacologically active ingredients whose simultaneous administration is useful.

The amount of compound of formula I in the pharmaceutical composition of the present invention may vary within a wide range as a function of known factors, for instance the type of disease to be treated, the severity of the disease, the bodyweight of the patient, the dosage form, the chosen route of administration, the number of daily administrations and the efficacy of the chosen compound of formula I. However, the optimum amount may be determined by a person skilled in the art in a simple and routine manner.

Typically, the amount of compound of formula I in the pharmaceutical composition of the present invention will be such that it ensures a level of administration of between 0.5 and 50 mg/kg/day and preferably between 2 and 20 mg/kg/day.

The dosage forms of the pharmaceutical composition of the present invention may be prepared according to techniques that are well known to pharmaceutical chemists, including mixing, granulation, compression, dissolution, sterilization and the like.

The examples that follow are given to illustrate the present invention without, however, limiting it.

In the examples that follow, the analytical characterizations of the compounds were performed using an HPLC-MS system (Gilson-ThermoFinnigan) using a Synergy Polar RP column (150 x 4.6; Phenomenex) or ODS3 column (150 x 4.6; GL Science).

### Example 1

2-(9-fluorenylmethoxycarbonyl)-2,3,4,9-tetrahydro-1H-pyrido[3,4-b]indolo-1-carboxylic acid

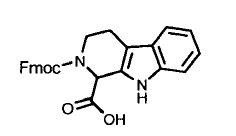

Tryptamine (8 g; 50 mmol) was dissolved in H₂O (155 ml) by addition of 6M HCl solution. A solution (11.20 ml) of glyoxalic acid (5.06 g; 68 mmol) in H₂O was then added to the reaction mixture.

The pH of the reaction mixture was then brought to about 3.5-4 with aqueous 3.5M KOH solution.

After stirring for 1 hour at room temperature, the formation of a white precipitate was noted.

The reaction mixture was filtered and the precipitate was then washed several times with H₂O and acetone. 1,2,3,4-Tetrahydro-beta-carboline (8.5 g; yield: about 78%) was thus obtained.

HPLC-MS (MH⁺ 217); LC-UV purity: 99.3% (λ = 220 nm) 98% (λ = 254 nm).

beta-Carboline (3 g; 14 mmol) and Na₂CO₃ (3 g; 28 mmol) were suspended in a solution of H₂O (180 ml) and dioxane (50 ml).

30 ml of a solution of Fmoc-Cl (4 g; 14 mmol) in dioxane at 0°C were added slowly to this mixture. After stirring for 24 hours at room temperature, the dioxane was removed by evaporation under reduced pressure.

The reaction mixture was diluted with diethyl ether and 2M HCl was added to pH 1.

The two phases were separated and the aqueous phase was washed several times with diethyl ether. The organic phases were combined, washed with brine and dried over Na₂SO₄. After filtration, the solvent was removed by evaporation, thus giving an oily product.

Subsequent treatment with pentane and filtration of the white solid obtained gave 5.93 g of the desired product.

HPLC-MS (M⁺ 439); LC-UV purity: 98% (λ = 20 nm), 96% (λ = 254 nm).

### Example 2

(1RS)-2-(4"-methoxy-4'-phenoxybenzenesulfonyl)-1,2,3,4-tetrahydronorarmano-1-carboxylic acid

The compound of Example 1 (65.8 mg; 0.15 mmol) in dry dichloromethane (4 ml) and DIEA (105 µl ; 0.6 mmol) were added to a 2-chlorotrityl resin (0.2 g; 0.24 mmol; loading 1.2 mmol/g) swollen beforehand in dry dichloromethane.

The mixture was reacted at room temperature for 12 hours.

Control of the loading on the resin (77.3%) was performed by measuring the absorbance at 301 nm of the piperidine-benzofulvene adduct obtained by treating a small aliquot of resin with piperidine at 20% in DMF.

All the resin was then treated with piperidine at 20% in DMF (two treatments of 20 minutes each) and washed several times with tetrahydrofuran/dichloromethane/tetrabutyl methyl ether and dried.

4-methoxy-4-phenoxybenzenesulfonyl chloride (130 mg; 0.45 mmol) in dry DCM (2.5 ml) was added to the functionalized resin (0.15 mmol) swollen beforehand in THF with 2 equivalents of DMAP for 1 hour.

After 18 hours at room temperature, the resin was treated with 500 µl of a 1:1:8 AcOH/TFE/DCM mixture. The liquid phase was collected in a flask and characterized analytically.

The best analytical conditions for the purification were found to be the following conditions: Inertsil C18 column, ODS-3; eluents: phase A H₂O + 0.1 % TFA and phase B CH₃CN + 0.1 % TFA; flow: 5 ml/min; gradient: 45%-70%; run time: 21 min; collection peak: 8 minutes.

After collection, the fractions were combined and freeze-dried to give 10.5 mg of pure compound.

HPLC-MS (MH⁺ 479). LC-UV purity: 99.2% (λ = 220 nm), 98% (λ = 254 nm).

### Example 3

(1RS)-2-(4'-phenylbenzenesulfonyl)-1,2,3,4-tetrahydro-norarmano-1-carboxylic acid

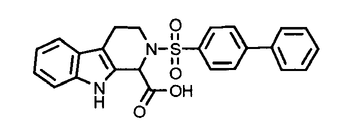

The resin derivatized (150 µmol) with the beta-carboline nucleus and deprotected from the Fmoc according to the process described in Example 2 above was allowed to swell in dry THF with 2 equivalents of DMAP (36.6 mg) for 1 hour. Benzenesulfonyl chloride (85.3 mg; 0.45 mmol) dissolved in dry DCM (1.5 ml) was then added.

After 18 hours at room temperature, the resin was treated with 500 µl of a 1:1:8 AcOH/TFE/DCM mixture. The liquid phase was collected in a flask and characterized analytically.

Purification was then performed under the following conditions: Inertsil C18 column, ODS-3; eluents: phase A H₂O + 0.08% TFA and phase B CH₃CN + 0.08% TFA; flow: 3 ml/min; gradient: 40%-95%; collection peak: 8 minutes.

After collection, the fractions were combined and freeze-dried to give 8.5 mg of pure compound.

HPLC-MS (MH⁺ 433). LC-UV purity: 96.3% (λ = 220 nm), 95.7% (λ = 254 nm).

### Example 4

(1 RS)-2-(4'-butoxybenzenesulfonyl)-1,2,3,4-tetrahydro-norarmano-1-carboxylic acid

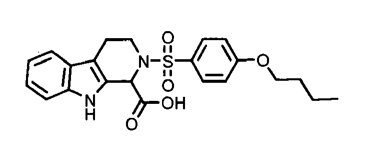

The resin derivatized (150 µmol) with the beta-carboline nucleus and deprotected with Fmoc according to the process described in Example 2 above was allowed to swell in dry THF with 2 equivalents of DMAP (36.6 mg) for 1 hour. 4'-butoxybenzenesulfonyl chloride (134 mg; 0.45 mmol) dissolved in dry DCM (1.5 ml) was then added.

After 18 hours at room temperature, the resin was treated with 500 µl of a 1:1:8 AcOH/TFE/DCM mixture. The liquid phase was collected in a flask and characterized analytically.

Purification was performed under the following conditions: Inertsil C18 column, ODS-3; eluents: phase A H₂O + 0.08% TFA and phase B CH₃CN + 0.08% TFA; flow: 3 ml/min; gradient: 40%-95%.

After collection, the fractions were combined and freeze-dried to give 7 mg of pure compound.

HPLC-MS (MH⁺ 429). LC-UV purity: 97% (λ = 220 nm), 98% (λ = 254 nm).

### Example 5

(1RS)-2-(4-methyl benzenesulfonyl)-1,2,3,4-tetrahydro-norarmano-1-carboxylic acid

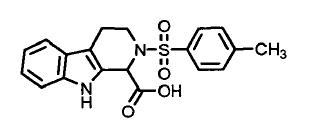

The resin derivatized (160 µmol) with the beta-carboline nucleus and deprotected from Fmoc according to the process described in Example 2 above was allowed to swell in dry THF with 2 equivalents of DMAP (36.6 mg) for 1 hour. Tosyl chloride (91.5 mg; 0.48 mmol) dissolved in dry DCM (1.5 ml) was then added.

After 18 hours at room temperature, the resin was treated with 500 µl of a 1:1:8 AcOH/TFE/DCM mixture. The liquid phase was collected in a flask and characterized analytically.

Purification was performed under the following conditions: Inertsil C18 column, ODS-3; eluents: phase A H₂O + 0.08% TFA and phase B CH₃CN + 0.08% TFA; flow: 3 ml/min; gradient: 35%-90%; run time: 20 min; collection peak: 8-9 minutes.

After collection, the fractions were combined and freeze-dried to give 6.7 mg of pure compound.

HPLC-MS (MH⁺ 371). LC-UV purity: 99% (λ = 220 nm), 98% (λ = 254 nm).

### Example 6

(1RS)-2-(2-naphthylsulfonyl)-1,2,3,4-tetrahydro-norarmano-1-carboxylic acid

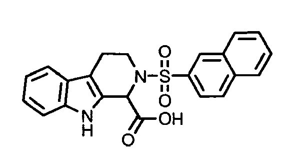

The resin derivatized (150 µmol) with the beta-carboline nucleus and deprotected from Fmoc according to the process described in Example 2 above was allowed to swell in dry THF with 2 equivalents of DMAP (36.6 mg) for 1 hour. 2-Naphthylsulfonyl chloride (102 mg; 0.45 mmol) dissolved in dry DCM (1.5 ml) was then added.

After 18 hours at room temperature, the resin was treated with 500 µl of a 1:1:8 AcOH/TFE/DCM mixture. The liquid phase was collected in a flask and characterized analytically.

Purification was performed under the following conditions: Inertsil C18 column, ODS-3; eluents: phase A H₂O + 0.08% TFA and phase B CH₃CN + 0.08% TFA; flow: 3 ml/min; gradient: 40%-95%.

After collection, the fractions were combined and freeze-dried to give 8.3 mg of pure compound.

HPLC-MS (MH⁺ 407). LC-UV purity: 94.6% (λ = 220 nm), 96% (λ = 254 nm).

### Example 7

(1 RS)-2-(4'-*t*-butylbenzenesulfonyl)-1,2,3,4-tetrahydro-norarmano-1-carboxylic acid

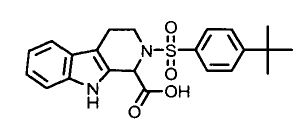

The resin derivatized (150 µmol) with the beta-carboline nucleus and deprotected from Fmoc according to the process described in Example 2 above was allowed to swell in dry THF with 2 equivalents of DMAP (36.6 mg) for 1 hour. 4-isopropylbenzenesulfonyl chloride (111.6 mg; 0.45 mmol) dissolved in dry DCM (1.5 ml) was then added.

After 18 hours at room temperature, the resin was treated with 500 µl of a 1:1:8 AcOH/TFE/DCM mixture. The liquid phase was collected in a flask and characterized analytically.

Purification was performed under the following conditions: Inertsil C18 column, ODS-3; eluents: phase A H₂O + 0.08% TFA and phase B CH₃CN + 0.08% TFA; flow: 3 ml/min; gradient: 40%-95%.

After collection, the fractions were combined and freeze-dried to give 6.4 mg of pure compound.

HPLC-MS (MH⁺ 413). LC-UV purity: 99% (λ = 220 nm), 98% (λ = 254 nm).

### Example 8

(1 RS)-2-(4"-chloro-4'-phenoxybenzenesulfonyl)-1,2,3,4-tetrahydronorarmano-1-carboxylic acid

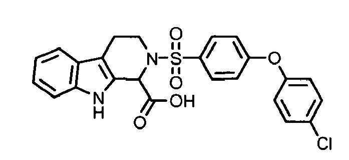

The resin derivatized (150 µmol) with the beta-carboline nucleus and deprotected from Fmoc according to the process described in Example 2 above was allowed to swell in dry THF with 2 equivalents of DMAP (36.6 mg) for 1 hour. 4"-chloro-4'-phenoxybenzenesulfonyl chloride (136 mg; 0.45 mmol) dissolved in dry DCM (1.5 ml) was then added.

After 18 hours at room temperature, the resin was treated with 500 µl of a 1:1:8 AcOH/TFE/DCM mixture. The liquid phase was collected in a flask and characterized analytically.

Purification was performed under the following conditions: Fenomenex C18 column; eluents: phase A H₂O + 0.1% TFA and phase B CH₃CN + 0.1% TFA; flow: 4 ml/min; gradient: 35%-70%; run time: 19 min; collection peak: 10 minutes.

After collection, the fractions were combined and freeze-dried to give 7.5 mg of pure compound.

HPLC-MS (MH⁺ 483). LC-UV purity: 98.2% (λ = 220 nm), 97% (λ = 254 mm).

### Example 9

(1 RS)-2-(4"-fluoro-4'phenoxybenzenesulfonyl)-1,2,3,4-tetrahydronorarmano-1-carboxylic acid

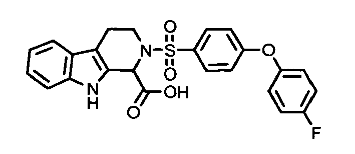

The resin derivatized (150 µmol) with the beta-carboline nucleus and deprotected from Fmoc according to the process described in Example 2 above was allowed to swell in dry THF with 2 equivalents of DMAP (36.6 mg) for 1 hour. 4"-fluoro-4'-phenoxybenzenesulfonyl chloride (128.7 mg; 0.45 mmol) dissolved in dry DCM (1.5 ml) was then added.

After 18 hours at room temperature, the resin was treated with 500 µl of a 1:1:8 AcOH/TFE/DCM mixture. The liquid phase was collected in a flask and characterized analytically.

Purification was performed under the following conditions: Inertsil C18 column, ODS-3; eluents: phase A H₂O + 0.08% TFA and phase B CH₃CN + 0.08% TFA; flow: 5 ml/min; gradient: 40%-70%; run time: 21 min; collection peak: 11 minutes.

After collection, the fractions were combined and freeze-dried to give approximately 7 mg of pure compound.

HPLC-MS (MH⁺ 467). LC-UV purity: 95.1 % (λ = 220 nm), 94% (λ = 254 nm).

### Example 10

(1R,S)-2-(4'-butoxybenzenesulfonyl)-1,2,3,4-tetrahydro-norarmano-N-hydroxy-1-carboxamide

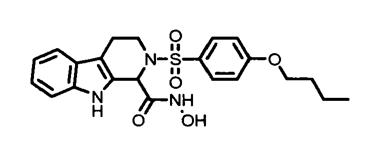

To increase the loading on the hydroxymethyl resin and to limit the side reactions on the particularly reactive beta-carboline nucleus, the fluoride loading method of Kaduk et al. (Letters in Peptide Science. 1996, 2, 285-288) was improved.

N-Fmoc-beta-carboline (263.1 mg; 0.6 mmol) was dissolved in dry DCM (3.5 ml) and dry DMF (500 µl). After adding DAST ((diethylamino)sulfur trifluoride) (95 µl; 0.72 mmol), the reaction mixture was stirred for 3 hours. The desired product was extracted with DCM/H₂O. The organic phases were combined, dried over Na₂SO₄ and evaporated to dryness.

The acyl fluoro compound thus obtained was dissolved in dry DMF (4 ml) and placed on 0.2 g of NovaSyn TGOH resin (0.3 mmol/g) which was swollen beforehand in dry DCM with 4-dimethylaminopyridine (DMAP) (0.7 mg; 6 µmol).

Monitoring of the loading on the resin was performed by measuring the absorbance at 301 nm of the piperidine-benzofulvene adduct obtained by treating a small aliquot of resin with piperidine at 20% in DMF.

After determining the loading of the resin (63%), it is treated with piperidine at 20% in DMF (two treatments of 20 minutes each), washed several times with THF/DCM/TBME and dried.

The resin (38 µmol) was then allowed to swell in THF with 2 equivalents of DMAP for 1 hour. 4-Butoxybenzene-1-sulfonyl chloride (75 mg, 0.3 mmol) dissolved in dry DCM was then added such that the dry THF/dry DCM ratio was 2 : 1 (3 ml of dry THF, 1.5 ml of dry DCM).

After 18 hours at room temperature, the resin was treated with NH₂OH at 50 wt% in H₂O (500 µl ; 8 mmol) in THF (3 ml) for 24 hours. The liquid phase was collected in a flask and characterized analytically.

Purification was performed under the following conditions: Inertsil C18 column, ODS-3; eluents: phase A H₂O + 0.08% TFA and phase B CH₃CN + 0.08% TFA; flow: 5 ml/min; gradient: 35%-70%; run time: 20 min; collection peak: 8 minutes.

After collection, the fractions were combined and freeze-dried to give 8 mg of pure compound.

HPLC-MS (MH⁺ 444). LC-UV purity: 98.3% (λ = 220 nm), 97% (λ = 254 nm).

### Example 11

(1R,S)-2-(4'biphenylsulfonyl)-1,2,3,4-tetrahydro-norarmano-N-hydroxy-1-carboxamide

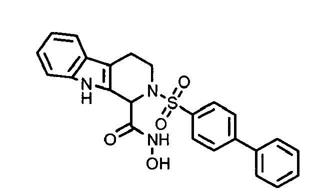

The resin derivatized (60 µmol) with the beta-carboline nucleus and deprotected from Fmoc according to the process described in Example 10 above was allowed to swell in THF with 2 equivalents of DMAP for 1 hour. Biphenylsulfonyl chloride (106 mg; 0.42 mmol) dissolved in dry DCM was then added such that the dry THF/dry DCM ratio was 2 : 1 (3 ml of dry THF, 1.5 ml of dry DCM).

After 18 hours at room temperature, the resin was treated with NH₂OH at 50 wt% in H₂O (750 µl; 12 mmol) in THF (3 ml) for 24 hours. The liquid phase was collected in a flask and characterized analytically.

Purification was performed under the following conditions: Inertsil C18 column, ODS-3; eluents: phase A H₂O + 0.08% TFA and phase B CH₃CN + 0.08% TFA; flow: 5 ml/min; gradient: 25%-70%; run time: 18 min; collection peak: 10 minutes.

After collection, the fractions were combined and freeze-dried to give 7 mg of pure compound.

HPLC-MS (MH⁺ 448). LC-UV purity: 93% (λ = 220 nm), 95% (λ = 254 nm).

### Example 12

(1 RS)-2-(4"-methoxy-4'-phenoxybenzenesulfonyl)-1,2,3,4-tetrahydronorarmano-N-hydroxy-1-carboxamide

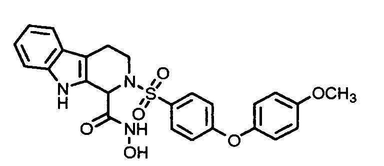

The resin derivatized (60 µmol) with the beta-carboline nucleus and deprotected from Fmoc according to the process described in Example 10 above was allowed to swell in THF with 2 equivalents of DMAP for 1 hour. 4"-Methoxy-4'-phenoxybenzenesulfonyl chloride (125.5 mg; 0.42 mmol) dissolved in dry DCM was then added such that the dry THF/dry DCM ratio was 2 : 1 (3 ml of dry THF, 1.5 ml of dry DCM).

After 18 hours at room temperature, the resin was treated with NH₂OH at 50 wt% in H₂O (750 µl; 12 mmol) in THF (3 ml) for 24 hours. The liquid phase was collected in a flask and characterized analytically.

Purification was performed under the following conditions: Inertsil C18 column, ODS-3; eluents: phase A H₂O + 0.1 % TFA and phase B CH₃CN + 0.1 % TFA; flow: 5 ml/min; gradient: 25%-70%; run time: 16 min; collection peak: 10.5 minutes.

After collection, the fractions were combined and freeze-dried to give 6.5 mg of pure compound.

HPLC-MS (MH⁺ 494). LC-UV purity: 93.2% (λ = 220 nm), 92% (λ = 254 nm).

### Example 13

(1 RS)-2-(4"-chloro-4'-phenoxybenzenesulfonyl)-1,2,3,4-tetrahydronorarmano-N-hydroxy-1-carboxamide

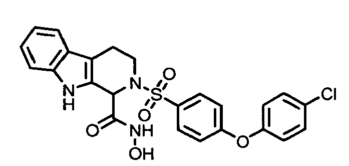

The resin derivatized (60 µmol) with the beta-carboline nucleus and deprotected from Fmoc according to the process described in Example 10 above was allowed to swell in THF with 2 equivalents of DMAP for 1 hour. 4"-chloro-4'-phenoxybenzenesulfonyl chloride (127.3 mg; 0.42 mmol) dissolved in dry DCM was then added such that the dry THF/dry DCM ratio was 2 : 1 (3 ml of dry THF, 1.5 ml of dry DCM).

After 18 hours at room temperature, the resin was treated with NH₂OH at 50 wt% in H₂O (750 µl; 12 mmol) in THF (3 ml) for 24 hours. The liquid phase was collected in a flask and characterized analytically.

Purification was performed under the following conditions: proteon C18 column; eluents: phase A H₂O + 0.1% TFA and phase B CH₃CN + 0.1% TFA; flow: 5 ml/min; gradient: 35%-70%; run time: 21 min; collection peak: 13.5 minutes.

After collection, the fractions were combined and freeze-dried to give 7.3 mg of pure compound.

HPLC-MS (MH⁺ 498). LC-UV purity: 99.4% (λ = 220 nm), 98% (λ = 254 nm).

### Example 14

(1RS)-2-(4"-fluoro-4'-phenoxybenzenesulfonyl)-1,2,3,4-tetrahydronorarmano-N-hydroxy-1-carboxamide

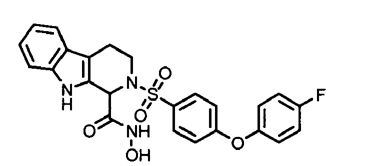

The resin derivatized (60 µmol) with the beta-carboline nucleus and deprotected from Fmoc according to the process described in Example 10 above was allowed to swell in THF with 2 equivalents of DMAP for 1 hour. 4"-fluoro-4'-phenoxybenzenesulfonyl chloride (120.4 mg; 0.42 mmol) dissolved in dry DCM was then added such that the dry THF/dry DCM ratio was 2 : 1 (3 ml of dry THF, 1.5 ml of dry DCM).

After 18 hours at room temperature, the resin was treated with NH₂OH at 50 wt% in H₂O (750 µl; 12 mmol) in THF (3 ml) for 24 hours. The liquid phase was collected in a flask and characterized analytically.

Purification was performed under the following conditions: Inertsil C18 column, ODS-3; eluents: phase A H₂O + 0.08% TFA and phase B CH₃CN + 0.08% TFA; flow: 5 ml/min; gradient: 35%-70%; run time: 16.5 min; collection peak: 9 minutes.

After collection, the fractions were combined and freeze-dried to give 6.5 mg of pure compound.

HPLC-MS (MH⁺ 482). LC-UV purity: 99% (λ = 220 nm), 98% (λ = 254 nm).

### Example 15

### Enzymatic tests

Determination of the modulatory activity on the proteolytic action of the various MMPs tested (MMP1, 2, 3, 8, 9, 12, 13 and 14) using the compounds of formula (I) according to the present invention was performed via fluorometry on a 96-well plate.

For comparative purposes, the activity of the following known compounds was also determined:
- 6-methoxy-1,2,3,4-tetrahydronorarmano-1-carboxylic acid (EP 0 891 187 B1);
- 2-[(4-methoxyphenyl)sulfonyl]-9-{[2-(2-morpholin-4-ylethylsulfanyl)ethylcarbamoyl]methyl}-2,3,4,9-tetrahydro-1H-β-carboline-(3R)-(N-hydroxy)carboxamide hydrochloride (US 6 066 633 A; Example 11);
- (3S)-N-hydroxy-4-(4-(pyrid-4-yloxyl)benzenesulfonyl)-2,2-dimethyltetrahydro-2H-1,4-thiazine-3-carboxamide (prinomastat).

The test compounds were dissolved in DMSO to a concentration of 5 mM or 50 mM. 5-fold or 10-fold (D5 or D10) serial dilutions of each compound were prepared so as to reach the nominal range of 5 assays on which the test was performed. Each of the dilutions was added to a well containing 150 µl of activated buffer (50 mM Tris/HCl; 0.1 M NaCl; 10 mM CaCl₂; 0.05% Brij 35) together with the enzyme on which the test was performed.

The concentration of the enzyme [in full-length form in the case of MMP2; in the form of the catalytic domains alone for the other MMPs] ranged between 0.5 nM and 3 nM based on the activity of the enzyme on the fluorogenic substrate used (7-methoxycoumarin-4-yl)-Pro-Leu-Gly-Leu-DPA-Ala-Arg-NH₂ (MCA-1) for MMP 1, 2, 3, 8, 9, 12 and 14) and MCA-Pro-(L-cyclohexylalanine)-Gly-(L-norvaline)-His-Ala-3-(2,4-dinitrophenyl)-L-2,3-diaminopropionyl)-NH₂ for MMP13) (Knight et al. 1992. FEBS Lett. 296, 263; Knauper et al. 1996, J. Biol. Chem. 271, 1544).

After incubation for one hour at 37°C of the compound with the enzyme, 50 µl of a solution of fluorogenic substrate were added to a final concentration of between 2 and 7 µM.

The fluorometric readings were then taken using a Varian Cary Eclipse spectrofluorometer regulated on the specific wavelength for the substrate used, at time T0 and after 3 hours of incubation at 37° (T3h).

From the analysis of the data obtained, the IC₅₀ values, the concentration of compound capable of inhibiting 50% of the enzymatic activity were calculated.

In the case of particularly active compounds, it was not possible to obtain an IC₅₀ value, given the intrinsic limit of the test that does not make it possible to evaluate the activity of derivatives that have a concentration that inhibits 50% of the enzymatic activity that is less than half the enzymatic concentration.

The results obtained are given in Table 1 below.

**Table 1**

| IC₅₀ values determined via the fluorimetric test | | | | | | | | |
|---|---|---|---|---|---|---|---|---|
| Compound | IC₅₀MMP2 (full enzyme) | IC₅₀ MMP9 (catalytic domain) | IC₅₀ MMP1 (catalytic domain) | IC₅₀ MMP3 (catalytic domain) | IC₅₀ MMP13 (catalytic domain) | IC₅₀ MMP8 (catalytic domain) | IC₅₀ MMP12 (catalytic domain) | IC₅₀ MMP14 (catalytic domain) |
| 2 | 0.6±0.1 µM | 2.2 ± 0.8 µM | > 50 µM | > 50µM | 15 ± 5 µM | 1.6 ± 0.2 µM | 0.5±0.1 µM | > 50 µM |
| 3 | 0.6 ± 0.8 µM | 7.5 ± 5 µM | > 50 µM | 11±1 µM | 0.16 ± 0.05 µM | 3±0.7 µM | 0.3±0.05 µM | 30 ± 2 µM |
| 4 | 0.68 ± 0.2 µM | 24 ± 1 µM | > 50 µM | | 2 ± 0.5 µM | 30.00 µM | 4.5 ± 1 µM | ND |
| 5 | 15.00 µM | 141.00 µM | ND | ND | ND | ND | ND | ND |
| 6 | 2.5 ± 0.1 µM | 62 ± 5 µM | ND | ND | ND | ND | ND | ND |
| 7 | > 125 µM | ND | ND | ND | ND | ND | ND | ND |
| 8 | 10±2 µM | 15±4 µM | >50 µM | >50 µM | 0.8 ± 0.2 µM | 10 ± 1 µM | 2±0.2 µM | > 50 µM |
| 9 | 28 ± 1 µM | 4 ± 1 µM | > 50 µM | > 50 µM | > 50 µM | 21 ± 2 µM | 2 ± 0.3 µM | > 50 µM |
| 10 | 7±2.5nM | 6±2nM | 20±2 µM | 4±1.5 µM | 2±0.5 µM | <0.5 nM | <0.5 nM | 50±11 nM |
| 11 | 14 ± 3 nM | 15 ± 2 nM | 3 ± 1 µM | 0.60 µM | 3 nM | 4.7 ± 0.5 nM | 4 ± 1.6 nM | ND |
| 12 | <0.3 nM | <0.5 nM | 0.35 ± 0.1 µM | 30 ± 10 µM | <0.5nM | <0.5nM | <0.5nM | 5 ± 1 nM |
| 13 | <0.3 nM | 0.63 ± 0.2 nM | >50 µM | 0.25 ± 0.05 µM | 2 ± 0.1 nM, | 12 ± 5 nM | 70 ± 30 mM | 30 ± 3nM |
| 14 | <0.3 nM | <0.5 nM | 0.34 ± 0.1 µM | 0.6 ± 0.2 µM | < 0.5 nM | < 0.5 nM | ND | 3.2 ± 0.1 nM |
| 6-methoxy-1,2,3,4-tetrahydronorarmano-1-carboxylic acid (EP 0 891 187 B1) | | | | | | 0.2 µM | | |
| US 006 066 633 A (Example 11) | 1.6 nM | 0.1 nM | | 3.1 nM | | | | |
| prinomastat | <0.3 nM | <0.5 nM | 7±1 nM | 1.4±0.4nM | <0.5 nM | <0.5 nM | <0.5 nM | |
| prinomastat* | 0.00005 ± 0.00002 | | 0.0083 ± 0.0008 | 0.0003 ± 0.0001 | 0.00003 ± 0.00001 | | | |

| | | | | | | | | |
|---|---|---|---|---|---|---|---|---|
| (*) IC₅₀ value for prinomastat according to Shalinsky et al. (Ann. NY Acad. Sci. 1999, 878, 236-270) | | | | | | | | |

## Claims

1. Arylsulfonamide of formula (I) 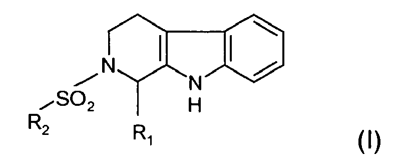 in which
R₁ is a carboxylic or hydroxamic group, and
R₂ is an aryl group chosen from the class comprising
- a naphthyl group and
- a phenyl group optionally substituted in the para position with
(a) a halogen atom,
(b) a linear or branched alkyl group containing from 1 to 5 carbon atoms,
(c) an alkoxy group in which the linear or branched alkyl group contains from 1 to 5 carbon atoms,
(d) a phenyl group which, in turn, is optionally substituted in the para position with a halogen atom, a linear or branched alkyl group containing from 1 to 5 carbon atoms, or an alkoxy group in which the linear or branched alkyl group contains from 1 to 5 carbon atoms, or
(e) a phenoxy group in which the phenyl group is optionally substituted in the para position with a halogen atom, a linear or branched alkyl group containing from 1 to 5 carbon atoms or an alkoxy group in which the linear or branched alkyl group contains from 1 to 5 carbon atoms;
stereoisomers thereof and, when R₁ is a carboxylic group, pharmaceutically acceptable base-addition salts thereof.

2. Arylsulfonamide according to Claim 1 in which the halogen is chlorine or fluorine.

3. Arylsulfonamide according to Claim 1 or 2 in which R₂ is phenyl, 2-naphthyl, 4'-methylphenyl, 4'-tert-butylphenyl, 4'-butoxyphenyl, biphenyl, 4"-methoxy-4'-phenoxyphenyl, 4"-chloro-4'-phenoxyphenyl or 4"-fluoro-4'-phenoxyphenyl.

4. Process for preparing an arylsulfonamide of formula (I) 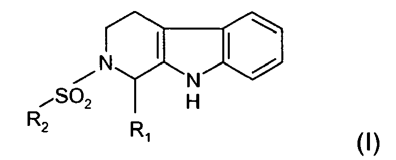 in which R₁ and R₂ have the meanings given in the preceding Claims 1 to 3,
and, when R₁ is a carboxylic group, the pharmaceutically acceptable base-addition salts thereof,
**characterized in that**:
(a)
(i) a compound of formula (II) 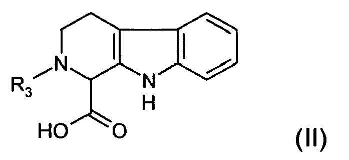 in which R₃ is a protective group,
is immobilized on a suitable acid-labile solid support,
(ii) the protective group R₃ is removed,
(iii) the deprotected product is reacted with a sulfonyl chloride of formula (III)
R₂-SO₂Cl (III)
in which R₂ has the meanings given above,
in the presence of a suitable acid acceptor,
to give a compound of formula (I) in which R₁ is a carboxylic group,
(iv) the compound of formula (I) thus obtained is removed from the solid support, and
(v)if desired, the compound of formula (I) is salified with a pharmaceutically acceptable base, or
(b)
(i) a compound of formula (II) is fluorinated to give a compound of formula (IV) 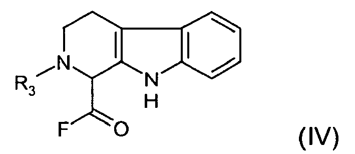 in which R₃ has the meaning given above,
(ii) the compound of formula (IV) thus obtained is immobilized on a suitable solid support,
(iii) the protective group R₃ is removed,
(iv) the deprotected product is reacted with a sulfonyl chloride of formula (III)
R₂-SO₂-Cl (III)
in which R₂ has the meanings given above in the presence of a suitable acid acceptor,
(v) the product thus obtained is removed from the solid support with NH₂OH to give a compound of formula (I) in which R₁ is a hydroxamic group.

5. Process according to Claim 4, in which the protective group R₃ is the fluorenylmethoxycarbonile group (Fmoc).

6. Process according to Claim 4 or 5, in which the acid-labile solid support used in step (a)(i) is a chlorotrityl resin.

7. Process according to any one of Claims 4 to 6, in which, in step (a)(i), the immobilization of the compound of formula (II) on the chlorotrityl resin is performed in the presence of diisopropylethylamine.

8. Process according to any one of Claims 4 to 7, in which, in steps (a)(ii) and (b)(iii), the removal of the Fmoc protective group is performed with piperidine at 20% in dimethylformamide.

9. Process according to any one of Claims 4 to 8, in which the reaction of step (a)(iii) is performed by reacting the deprotected product with a moderate excess of sulfonyl chloride of formula (III).

10. Process according to any one of Claims 4 to 9, in which step (a)(iv) is performed using a 1:1:8 acetic acid/trifluoroethanol/dichloromethane mixture.

11. Process according to any one of Claims 4 to 10, in which the solid support used in step (b)(ii) is a hydroxymethyl resin.

12. Process according to any one of Claims 4 to 11, in which the reaction of step (b)(iv) is performed by reacting the deprotected product with an excess of sulfonyl chloride of formula (III).

13. Process according to any one of Claims 4 to 12, in which step (b)(v) is performed with NH₂OH as an aqueous 50% solution.

14. Pharmaceutical composition comprising an arylsulfonamide of formula (I) 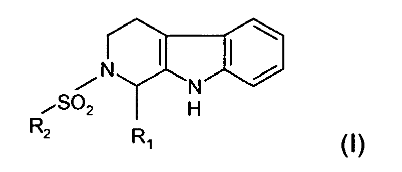 in which
R₁ is a carboxylic or hydroxamic group, and
R₂ is an aryl group chosen from the class comprising
- a naphthyl group and
- a phenyl group optionally substituted in the para position with
(a) a halogen atom,
(b) a linear or branched alkyl group containing from 1 to 5 carbon atoms,
(c) an alkoxy group in which the linear or branched alkyl group contains from 1 to 5 carbon atoms,
(d) a phenyl group which, in turn, is optionally substituted in the para position with a halogen atom, a linear or branched alkyl group containing from 1 to 5 carbon atoms, or an alkoxy group in which the linear or branched alkyl group contains from 1 to 5 carbon atoms, or
(e) a phenoxy group in which the phenyl group is optionally substituted in the para position with a halogen atom, a linear or branched alkyl group containing from 1 to 5 carbon atoms or an alkoxy group in which the linear or branched alkyl group contains from 1 to 5 carbon atoms.
a stereoisomer thereof or, when R₁ is a carboxylic group, a pharmaceutically acceptable base-addition salt thereof,
and at least one physiologically acceptable vehicle.

15. Pharmaceutical composition according to Claim 14, in which the halogen is chlorine or fluorine.

## Patentansprüche

1. Arylsulfonamid der Formel (I) 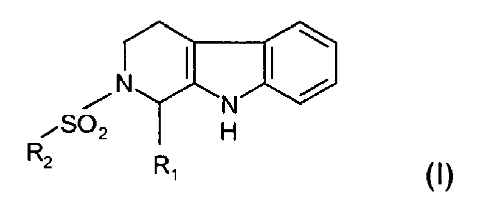 bei der R₁ eine Carboxyl- oder Hydroxamgruppe ist, und
R₂ eine Arylgruppe ist, die ausgewählt ist aus der Klasse mit
- einer Naphthylgruppe und
- einer Phenylgruppe, die wahlweise in der para-Stellung mit
(a) einem Halogenatom,
(b) einer linearen oder verzweigten Alkylgruppe, die 1 bis 5 Kohlenstoffatome aufweist,
(c) einer Alkoxygruppe, bei der die lineare oder verzweigte Alkylgruppe 1 bis 5 Kohlenstoffatome aufweist,
(d) einer Phenylgruppe, die der Reihe nach in der para-Stellung wahlweise mit einem Halogenatom, einer linearen oder verzweigten Alkylgruppe, die 1 bis 5 Kohlenstoffatome aufweist, oder einer Alkoxygruppe, bei der die lineare oder verzweigte Alkylgruppe 1 bis 5 Kohlenstoffatome enthält, substituiert ist, oder
(e) einer Phenoxygruppe, bei der die Phenylgruppe in der para-Stellung wahlweise mit einem Halogenatom, einer linearen oder verzweigten Alkylgruppe, die 1 bis 5 Kohlenstoffatome enthält, oder einer Alkoxygruppe, bei der die lineare oder verzweigte Alkylgruppe 1 bis 5 Kohlenstoffatome aufweist, substituiert ist,
Stereoisomeren davon und, wenn R₁ eine Carboxylgruppe ist, pharmazeutisch verträglichen Base-Additions-Salzen davon.

2. Arylsulfonamid nach Anspruch 1, bei dem das Halogen Chlor oder Fluor ist.

3. Arylsulfonamid nach Anspruch 1 oder 2, bei dem R₂ Phenyl, 2-Naphthyl, 4'-Methylphenyl, 4'-tert-Butylphenyl, 4'-Butoxyphenyl, Biphenyl, 4"-Methoxy-4'-phenoxyphenyl, 4"-Chlor-4'-phenoxyphenyl oder 4"-Fluor-4'-phenoxyphenyl ist.

4. Verfahren zum Herstellen eines Arylsulfonamids der Formel (I) 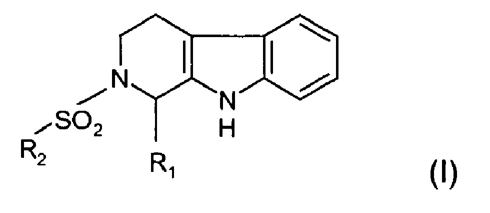 bei der R₁ und R₂, die in den vorstehenden Ansprüchen 1 und 3 angegebenen Bedeutungen haben, und wenn R₁ eine Carboxylgruppe ist, die pharmazeutisch verträglichen Base-Additions-Salze davon,
**dadurch gekennzeichnet, dass**
(a)
(i) eine Verbindung der Formel (II) 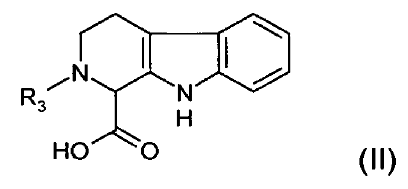 bei der R₃ eine Schutzgruppe ist,
auf einem geeigneten säurelabilen festen Träger immobilisiert wird,
(ii) die Schutzgruppe R₃ entfernt wird,
(iii) das entschützte Produkt mit einem Sulfonylchlorid der Formel (III)
**R₂-SO₂Cl** (III)
bei dem R₂ die oben angegebenen Bedeutungen hat,
in Gegenwart eines Säureakzeptors zur Reaktion gebracht wird, um eine Verbindung (I), bei der R₁ eine Carboxylgruppe ist, zu erhalten,
(iv) die so erhaltene Verbindung der Formel (I) von dem festen Träger entfernt wird, und
(v) falls gewünscht, die Verbindung der Formel (I) mit einer pharmazeutisch verträglichen Base in ein Salz überführt wird, oder
(b)
(i) eine Verbindung der Formel (II) fluoriert wird, um eine Verbindung der
Formel (IV) 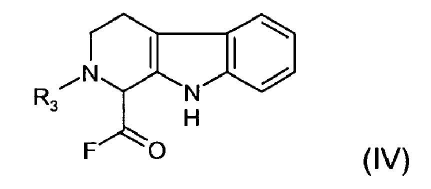 zu erhalten, bei der R₃ die oben angegebene Bedeutung hat,
(ii) die so erhaltene Verbindung der Formel (IV) auf einem geeigneten festen Träger immobilisiert wird,
(iii) die Schutzgruppe R₃ entfernt wird,
(iv) das entschützte Produkt mit einem Sulfonylchlorid der Formel (III)
**R₂-SO₂Cl** (III)
in Gegenwart eines geeigneten Säureakzeptors zur Reaktion gebracht wird, bei der R₂ die oben angegebenen Bedeutungen hat,
(v) das so erhaltene Produkt von dem festen Träger mit NH₂OH entfernt wird, um eine Verbindung der Formel (I) zu erhalten, bei der R₁ eine Hydroxamgruppe ist.

5. Verfahren nach Anspruch 4, bei dem die Schutzgruppe R₃ die Fluorenylmethoxycarbonylgruppe (Fmoc) ist.

6. Verfahren nach Anspruch 4 oder 5, bei dem der in Schritt (a)(i) verwendete säurelabile feste Träger ein Chlorotritylharz ist.

7. Verfahren nach einem der Ansprüche 4 bis 6, bei dem in Schritt (a)(i) die Immobilisierung der Verbindung der Formel (II) auf dem Chlorotritylharz in Gegenwart von Diisopropylethylamin durchgeführt wird.

8. Verfahren nach einem der Ansprüche 4 bis 7, bei dem in den Schritten (a)(ii) und (b)(iii) die Entfernung der Fmoc-Schutzgruppe mit 20%-igem Piperidin in Dimethylformamid durchgeführt wird.

9. Verfahren nach einem der Ansprüche 4 bis 8, bei dem die Reaktion von Schritt (a)(iii) durch Umsetzen des entschützten Produktes mit einem mäßigen Überschuss an Sulfonylchlorid der Formel (III) durchgeführt wird.

10. Verfahren nach einem der Ansprüche 4 bis 9, bei dem Schritt (a)(iv) unter Verwendung einer 1:1:8 Essigsäure/Trifluorethanol/Dichlormethan-Mischung durchgeführt wird.

11. Verfahren nach einem der Ansprüche 4 bis 10, bei dem der in Schritt (b)(ii) verwendete feste Träger ein Hydroxymethylharz ist.

12. Verfahren nach einem der Ansprüche 4 bis 11, bei dem die Reaktion von Schritt (b)(iv) durch Umsetzen des entschützten Produktes mit einem Überschuss an Sulfonylchlorid der Formel (III) durchgeführt wird.

13. Verfahren nach einem der Ansprüche 4 bis 12, bei dem Schritt (b)(v) mit NH₂OH als eine wässrige 50%-Lösung durchgeführt wird.

14. Pharmazeutische Zusammensetzung mit einem Arylsulfonamid der Formel (I) 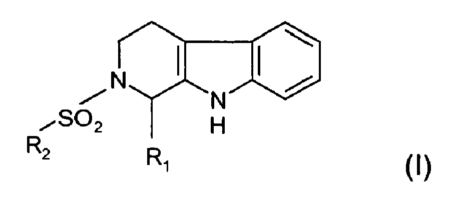 bei der
R₁ eine Carboxyl- oder Hydroxamgruppe ist, und
R₂ eine Arylgruppe ist, die ausgewählt ist aus der Klasse mit
- einer Naphthylgruppe und
- einer Phenylgruppe, die wahlweise in der para-Stellung substituiert ist, mit
(a) einem Halogenatom,
(b) einer linearen oder verzweigten Alkylgruppe, die 1 bis 5 Kohlenstoffatome enthält,
(c) einer Alkoxygruppe, bei der die lineare oder verzweigte Alkylgruppe 1 bis 5 Kohlenstoffatome enthält,
(d) einer Phenylgruppe, die der Reihe nach wahlweise in der para-Stellung mit einem Halogenatom, einer linearen oder verzweigten Alkylgruppe, die 1 bis 5 Kohlenstoffatome enthält, oder einer Alkoxygruppe, bei der die lineare oder verzweigte Alkylgruppe 1 bis 5 Kohlenstoffatome enthält, oder
(e) einer Phenoxygruppe, bei der die Phenylgruppe wahlweise in der para-Stellung mit einem Halogenatom, einer linearen oder verzweigten Alkylgruppe, die 1 bis 5 Kohlenstoffatome enthält, oder einer Alkoxygruppe, in der die lineare oder verzweigte Alkylgruppe 1 bis 5 Kohlenstoffatome enthält, substituiert ist,
einem Stereoisomer davon oder wenn R₁ eine Carboxylgruppe ist, einem pharmazeutisch verträglichen Base-Additions-Salz davon,
und zumindest einem physiologisch verträglichen Bindemittel.

15. Pharmazeutische Zusammensetzung nach Anspruch 14, bei der das Halogen Chlor oder Fluor ist.

## Revendications

1. Arylsulfonamide de formule (I) 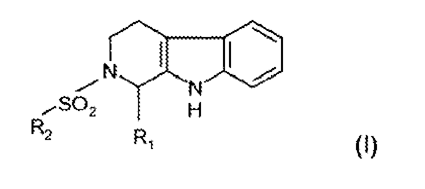 dans laquelle
R₁ est un groupe carboxylique ou hydroxamique, et
R₂ est un groupe aryle choisi dans la classe comprenant
- un groupe naphtyle et
- un groupe phényle facultativement substitué en position para par
(a) un atome d'halogène,
(b) un groupe alkyle linéaire ou ramifié contenant de 1 à 5 atomes de carbone,
(c) un groupe alcoxy dans lequel le groupe alkyle linéaire ou ramifié contient de 1 à 5 atomes de carbone,
(d) un groupe phényle qui est lui-même facultativement substitué en position para par un atome d'halogène, un groupe alkyle linéaire ou ramifié contenant de 1 à 5 atomes de carbone, ou un groupe alcoxy dans lequel le groupe alkyle linéaire ou ramifié contient de 1 à 5 atomes de carbone, ou
(e) un groupe phénoxy dans lequel le groupe phényle est facultativement substitué en position para par un atome d'halogène, un groupe alkyle linéaire ou ramifié contenant de 1 à 5 atomes de carbone, ou un groupe alcoxy dans lequel le groupe alkyle linéaire ou ramifié contient de 1 à 5 atomes de carbone ;
ses stéréoisomères et, lorsque R₁ est un groupe carboxylique, ses sels d'addition de base pharmaceutiquement acceptables.

2. Arylsulfonamide selon la revendication 1, dans lequel l'halogène est du chlore ou du fluor.

3. Arylsulfonamide selon la revendication 1 ou 2, dans lequel R₂ est un groupe phényle, 2-naphtyle, 4'-méthylphényle, 4'-tert-butylphényle, 4'-butoxyphényle, biphényle, 4"-méthoxy-4'-phénoxyphényle, 4"-chloro-4'-phénoxyphényle ou 4"-fluoro-4'-phénoxyphényle.

4. Procédé de préparation d'un arylsulfonamide de formule (I) 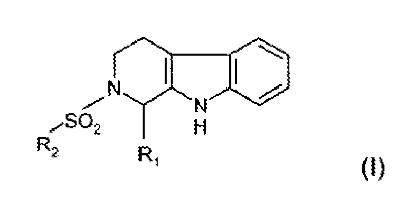 dans laquelle R₁ et R₂ ont les significations données dans les revendications 1 à 3 précédentes,
et, lorsque R₁ est un groupe carboxylique, des sels d'addition de base pharmaceutiquement acceptables de celui-ci,
**caractérisé en ce que** :
(a)
(i) un composé de formule (II) 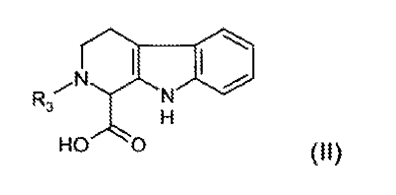 dans laquelle R₃ est un groupe protecteur, est immobilisé sur un support solide labile aux acides approprié,
(ii) le groupe protecteur R₃ est éliminé,
(iii) le produit déprotégé réagit avec un chlorure de sulfonyle de formule (III)
R₂-SO₂Cl (III)
dans laquelle R₂ a la signification donnée ci-dessus,
en présence d'un accepteur d'acide approprié,
pour donner un composé de formule (I) dans laquelle R₁ est un groupe carboxylique,
(iv) le composé de formule (I) ainsi obtenu est éliminé du support solide, et
(v) le cas échéant, le composé de formule (I) est salifié avec une base pharmaceutiquement acceptable, ou
(b)
(i) un composé de formule (II) est fluoré pour donner un composé de formule (IV) 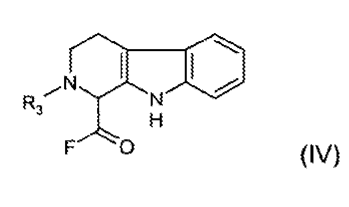 dans laquelle R₃ a la signification donnée ci-dessus,
(ii) le composé de formule (IV) ainsi obtenu est immobilisé sur un support solide approprié,
(iii) le groupe protecteur R₃ est éliminé,
(iv) le produit déprotégé réagit avec un chlorure de sulfonyle de formule (III)
R₂-SO₂Cl (III)
dans laquelle R₂ a la signification donnée ci-dessus,
en présence d'un accepteur d'acide approprié,
(v) le produit ainsi obtenu est éliminé du support solide avec NH₂OH pour donner un composé de formule (I) dans laquelle R₁ est un groupe hydroxamique.

5. Procédé selon la revendication 4, dans lequel le groupe protecteur R₃ est le groupe fluorénylméthoxycarbonile (Fmoc).

6. Procédé selon la revendication 4 ou 5, dans lequel le support solide labile aux acides utilisé dans l'étape (a)(i) est une résine de chlorotrityle.

7. Procédé selon l'une quelconque des revendications 4 à 6, dans lequel, dans l'étape (a)(i), l'immobilisation du composé de formule (II) sur la résine de chlorotrityle est effectuée en présence de diisopropyléthylamine.

8. Procédé selon l'une quelconque des revendications 4 à 7, dans lequel, dans les étapes (a)(ii) et (b)(iii), l'élimination du groupe protecteur Fmoc est effectuée avec de la pyridine à 20 % dans du diméthylformamide.

9. Procédé selon l'une quelconque des revendications 4 à 8, dans lequel, la réaction de l'étape (a)(iii) est effectuée en faisant réagir le produit déprotégé avec un excès modéré de chlorure de sulfonyle de formule (III).

10. Procédé selon l'une quelconque des revendications 4 à 9, dans lequel, l'étape (a)(iv) est effectuée au moyen d'un mélange acide acétique/trifluoroéthanol/dichlorométhane à 1 : 1 : 8.

11. Procédé selon l'une quelconque des revendications 4 à 10, dans lequel, le support solide utilisé dans l'étape (b)(ii) est une résine d'hydroxyméthyle.

12. Procédé selon l'une quelconque des revendications 4 à 11, dans lequel, la réaction de l'étape (b)(iv) est effectuée en faisant réagir le produit déprotégé avec un excès de chlorure de sulfonyle de formule (III).

13. Procédé selon l'une quelconque des revendications 4 à 12, dans lequel, l'étape (b)(v) est effectuée avec du NH₂OH sous forme de solution aqueuse à 50 %.

14. Composition pharmaceutique comprenant un arylsulfonamide de formule (I) 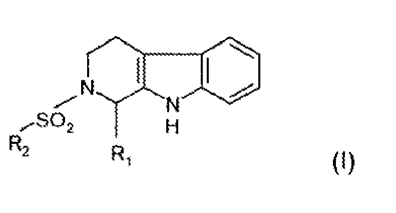 dans laquelle
R₁ est un groupe carboxylique ou hydroxamique, et
R₂ est un groupe aryle choisi dans la classe comprenant
- un groupe naphtyle et
- un groupe phényle facultativement substitué en position para par
(a) un atome d'halogène,
(b) un groupe alkyle linéaire ou ramifié contenant de 1 à 5 atomes de carbone,
(c) un groupe alcoxy dans lequel le groupe alkyle linéaire ou ramifié contient de 1 à 5 atomes de carbone,
(d) un groupe phényle qui est lui-même facultativement substitué en position para par un atome d'halogène, un groupe alkyle linéaire ou ramifié contenant de 1 à 5 atomes de carbone, ou un groupe alcoxy dans lequel le groupe alkyle linéaire ou ramifié contient de 1 à 5 atomes de carbone, ou
(e) un groupe phénoxy dans lequel le groupe phényle est facultativement substitué en position para par un atome d'halogène, un groupe alkyle linéaire ou ramifié contenant de 1 à 5 atomes de carbone, ou un groupe alcoxy dans lequel le groupe alkyle linéaire ou ramifié contient de 1 à 5 atomes de carbone ;
ses stéréoisomères et, lorsque R₁ est un groupe carboxylique, ses sels d'addition de base pharmaceutiquement acceptables,
et au moins un véhicule physiologiquement acceptable.

15. Composition pharmaceutique selon la revendication 14, dans laquelle l'halogène est un atome de chlore ou de fluor.
